# FASCICULE DE BREVET EUROPEEN

(11) **EP 1 403 277 B1**
(45) Date de publication et mention de la délivrance du brevet: **05.10.2005**
(21) Numéro de dépôt: 03290486.4
(22) Date de dépôt: 28.02.2003
(51) Int. Cl.: C07K 5/06, C07D 209/42, C07K 5/02

(54) **Nouveau procédé de synthèse de dérives de l'acide (2S, 3aS, 7aS)-1-((S)-alanyl)-octahydro-1H-indole-2-carboxylique, et application à la synthèse du perindopril**
Verfahren zur Synthese von (2S,3aS,7aS)-1-((S)-Alanyl)-octahydro-1H-indol-2-carbonsäurederivaten und Verwendung in der Synthese von Perindopril
Method for synthesis of (2S,3aS,7aS)-1-((S)-alanyl)-octahydro-1H-indole-2-carboxylic acid derivatives and use in the synthesis of perindopril

(43) Date de publication de la demande: 31.03.2004
(73) Titulaire: Les Laboratoires Servier, 92415 Courbevoie Cédex (FR)
(72) Inventeur: Dubuffet, Thiery, 76210 Bolbec (FR); Langlois, Pascal, 76210 Saint Jean de la Neuville (FR)

(56) Documents cités:
- WO-A-01/58868
- WO-A-03/016336
- VINCENT M ET AL: "Stereoselective Synthesis of a New Perhydroindole Derivative of Chiral Iminodiacid, a Potent Inhibitor of Agiotensin Converting Enzyme" TETRAHEDRON, ELSEVIER SCIENCE PUBLISHERS, AMSTERDAM, NL, vol. 23, no. 16, 1982, pages 1677-1680, XP002155080 ISSN: 0040-4020

## Description

La présente invention concerne un procédé de synthèse des composés de formule (I) : 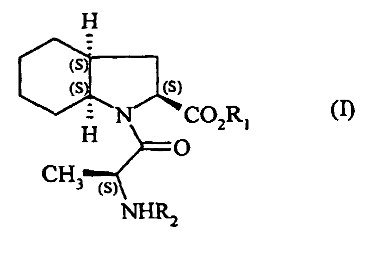 dans laquelle R₁ représente un atome d'hydrogène ou un groupement alkyle (C₁-C₆) linéaire ou ramifié ou benzyle, et R₂ représente un groupement protecteur de la fonction amino,
et leur application à la synthèse du perindopril de formule (II) : 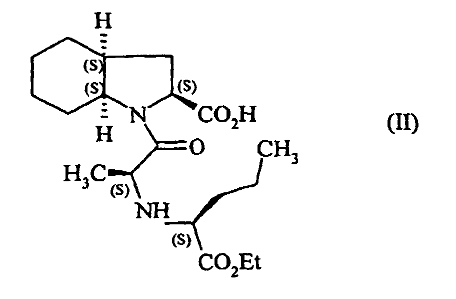 et de ses sels pharmaceutiquement acceptables.

Le perindopril, ainsi que ses sels pharmaceutiquement acceptables, et plus particulièrement son sel de tert-butylamine, possèdent des propriétés pharmacologiques intéressantes.
Leur principale propriété est d'inhiber l'enzyme de conversion de l'angiotensine I (ou kininase II), ce qui permet d'une part d'empêcher la transformation du décapeptide angiotensine I en octapeptide angiotensine II (vasoconstricteur), et d'autre part de prévenir la dégradation de la bradykinine (vasodilatateur) en peptide inactif.

Ces deux actions contribuent aux effets bénéfiques du perindopril dans les maladies cardiovasculaires, tout particulièrement l'hypertension artérielle et l'insuffisance cardiaque.

Le perindopril, sa préparation et son utilisation en thérapeutique ont été décrits dans le brevet européen EP 0 049 658.

Compte-tenu de l'intérêt pharmaceutique de ce composé, il était important de pouvoir y accéder avec un procédé de synthèse performant, conduisant au perindopril avec un bon rendement et avec une excellente pureté, à partir de matières premières bon marché et aisément accessibles.

La demande de brevet EP 1 256 590 décrit déjà un procédé de préparation des composés de formule (I).
Toutefois, celui-ci présente l'inconvénient d'utiliser comme matière première un ester de l'acide (2S)-dihydroindole-2-carboxylique, qui n'est pas commercial et dont la préparation nécessite plusieurs étapes de synthèse (dont une étape de résolution) à partir de l'acide indole-2-carboxylique.

La demanderesse a présentement mis au point un nouveau procédé de synthèse des dérivés de formule (I) qui présente l'avantage d'utiliser comme seules sources de chiralité l'alanine, matière première naturelle et donc peu coûteuse, et un dérivé aisément accessible de la sérine.

Plus spécifiquement, la présente invention concerne un procédé de synthèse industrielle du composé de formule (I) : 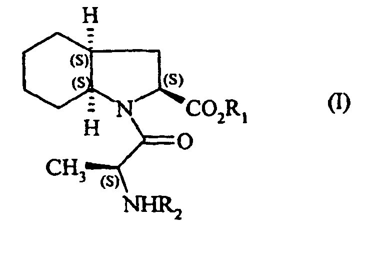 dans laquelle R₁ représente un atome d'hydrogène ou un groupement alkyle (C₁-C₆) linéaire ou ramifié ou benzyle, et R₂ représente un groupement protecteur de la fonction amine,
caractérisé en ce que l'on met en réaction la 1-(1-cyclohexén-1-yl)-pyrrolidine de formule (III) : 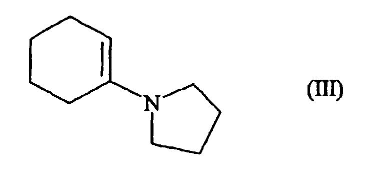 avec le dérivé de la sérine de formule (IV) : 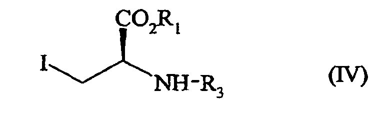 dans laquelle R₁ est tel que défini dans la formule (I), et R₃ représente un groupement protecteur de la fonction amine,
pour conduire au composé de formule (V) : 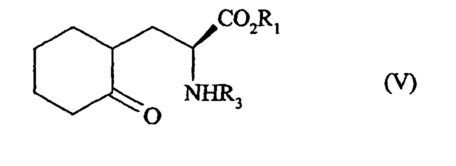 dans laquelle R₁ et R₃ sont tels que définis précédemment,
dont on déprotège la fonction amine avant d'en effectuer la cyclisation suivie de la déshydratation, pour conduire au composé de formule (VI) : 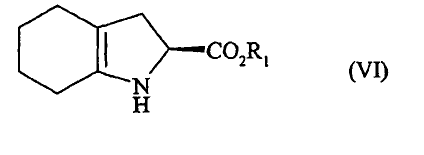 dans laquelle R₁ est tel que défini précédemment,
que l'on met en réaction avec le dérivé de l'alanine de formule (VII) : 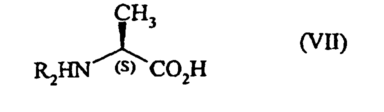 dans laquelle R₂ est tel que défini dans la formule (I),
dans un solvant organique tel que, par exemple, le tétrahydrofurane ou l'acétate d'éthyle, en présence d'une quantité de dicyclohexylcarbodiimide comprise entre 1 et 1,2 mole par mole de composé de formule (VI) utilisé, d'une quantité de triéthylamine comprise entre 1 et 1,2 mole par mole de composé de formule (VI) utilisé, et en présence éventuelle de 1-hydroxybenzotriazole,
à une température comprise entre 20 et 50°C,
pour conduire après isolement puis recristallisation au composé de formule (VIII) : 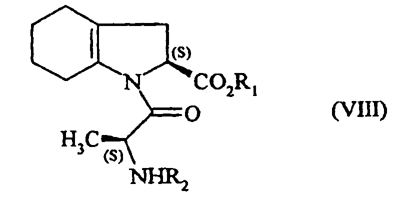 dans laquelle R₁ et R₂ sont tels que définis précédemment,
que l'on hydrogène en présence d'un catalyseur tel que, par exemple, le palladium, le platine, le rhodium ou le nickel,
sous une pression d'hydrogène comprise entre 1 et 30 bars, de préférence entre 1 et 10 bars , pour conduire, après éventuelle déprotection ou reprotection de la fonction acide, au composé de formule (I).

Le composé de formule (I) ainsi obtenu est ensuite soumis, le cas échéant, à une réaction de déprotection des fonctions acide et amine, suivie d'une réaction de couplage, soit avec le 2-oxo-pentanoate d'éthyle dans des conditions d'amination réductrice,
soit avec un composé de formule (IX) : 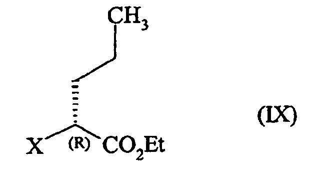 dans laquelle X représente un groupement partant choisi parmi atome d'halogène,

-O-SO₂CH₃

et 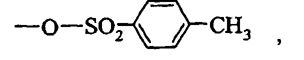 pour conduire au perindopril optiquement pur, que l'on transforme, si on le souhaite, en un sel pharmaceutiquement acceptable tel que le sel de tert-butylamine.

L'exemple ci-dessous illustre l'invention, mais ne la limite en aucune façon.

### EXEMPLE : Acide (2S, 3aS, 7aS)-1-{(2S)-2[(tert-butyloxycarbonyl)-amino]-propionyl}-octahydro-1H-indole-2-carboxylique

### Stade A : (2S)-2-[(Tert-butyloxycarbonyl)-amino]-3-(2-oxocyclohexyl)-propanoate de benzyle

Dans un réacteur équipé d'une colonne à reflux, placer 200 g de 1-(1-cyclohexén-1-yl)-pyrrolidine, 535 g de (25)-2-[(tert-butyloxycarbonyl)-amino]-3-iodopropanoate de benzyle et 1,5 l d'acétonitrile.
Amener au reflux pendant 1 h, puis ramener le mélange à température ambiante. Après évaporation du solvant, ajouter 2 l d'eau, puis de l'acide acétique. Extraire par l'acétate d'éthyle et évaporer à sec.
Le (2S)-2-[(tert-butyloxycarbonyl)-amino]-3-(2-oxocyclohexyl)-propanoate de benzyle est ainsi obtenu avec un rendement de 80 %.

### Stade B : (2S)-2-Amino-3-(2-oxocyclohexyl)-propanoate de benzyle

Dans un réacteur, placer 200 g du composé obtenu dans le stade précédent, 1,5 l de dichlorométhane et 60 g d'acide trifluoroacétique. Après 1 h30 d'agitation à température ambiante, ajouter 2 l d'une solution saturée d'hydrogénocarbonate de sodium. Extraire par le dichlorométhane et évaporer à sec.
Le (2S)-2-amino-3-(2-oxocyclohexyl)-propanoate de benzyle est ainsi obtenu avec un rendement de 90 %.

### Stade C : (2S)-2,3,4,5,6,7-Hexahydro-1H-indole-2-carboxylate de benzyle

Dans un réacteur, chauffer à reflux 200 g du composé obtenu dans le stade précédent, 13,8 g d'acide p-toluènesulfonique et 1 l de toluène en éliminant l'eau formée par entraînement azéotropique. Lorsqu'il ne décante plus d'eau, évaporer le toluène.
Le (2S)-2,3,4,5,6,7-hexahydro-1*H*-indole-2-carboxylate de benzyle est ainsi obtenu avec un rendement sur brut de 97 %.

### Stade D : (2S)-1-{(2S)-2-[(Tert-butyloxycarbonyl)-amino]-propionyl}-2,3,4,5,6,7-hexahydro-1H-indole-2-carboxylate de benzyle

Dans un réacteur sous agitation sont introduits 200 g du composé obtenu dans le stade précédent, 65 g de triéthylamine, 2 l de tétrahydrofurane puis, après 10 mn d'agitation à température ambiante, 123 g de N-[tert-butyloxycarbonyl]-(*S*)-alanine, et 130 g de dicylohexylcarbodiimide. Le mélange hétérogène est ensuite agité à température ambiante pendant 6 heures, puis il est refroidi à 0°C et filtré.
Le filtrat est ensuite lavé, puis recristallisé dans un mélange hexane/acétate d'éthyle 10/1 pour conduire au produit attendu avec un rendement de 81% et une pureté chimique de 98%.

### Stade E : Acide (2S, 3aS, 7aS)-1-{(2S)-2[(tert-butyloxycarbonyl)-amino]-propionyl}-octahydro-1H-indole-2-carboxylique

Dans un hydrogénateur, placer 200 g du composé obtenu dans le stade précédent, en solution dans l'acide acétique, puis 5 g de Pt/C à 10 %. Hydrogéner sous pression de 5 bars à température ambiante, jusqu'à absorption de la quantité théorique d'hydrogène.
Eliminer le catalyseur par filtration, puis refroidir entre 0 et 5°C et récolter le solide obtenu par filtration. Laver le gâteau et le sécher jusqu'à poids constant.
L'acide (2S, 3aS, 7aS)-1-{(2S)-2[(tert-butyloxycarbonyl)-amino]-propionyl}-octahydro-1*H*-indole-2-carboxylique est ainsi obtenu avec un rendement de 87 % et une pureté énantiomérique de 99 %.

## Revendications

1. Procédé de synthèse des composés de formule (I) : 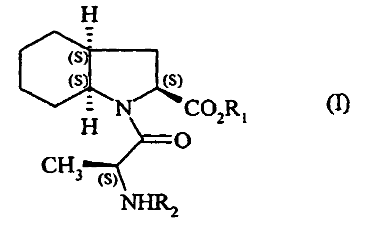 dans laquelle R₁ représente un atome d'hydrogène ou un groupement alkyle (C₁-C₆) linéaire ou ramifié ou benzyle, et R₂ représente un groupement protecteur de la fonction amine,
**caractérisé en ce que** l'on met en réaction la 1-(1-cyclohexén-1-yl)-pyrrolidine de formule (III) : 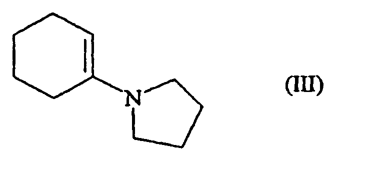 avec le dérivé de la sérine de formule (IV) : 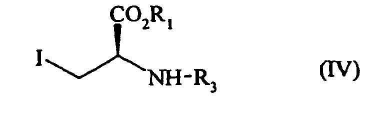 dans laquelle R₁ est tel que défini dans la formule (I), et R₃ représente un groupement protecteur de la fonction amine,
pour conduire au composé de formule (V) : 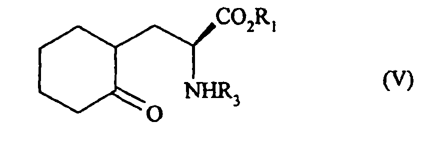 dans laquelle R₁ et R₃ sont tels que définis précédemment,
dont on déprotège la fonction amine avant d'en effectuer la cyclisation suivie de la déshydratation, pour conduire au composé de formule (VI) : 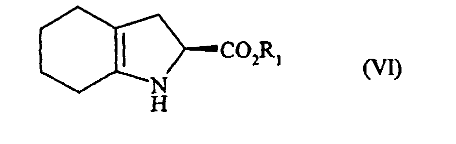 dans laquelle R₁ est tel que défini précédemment,
que l'on met en réaction avec le dérivé de l'alanine de formule (VII) : 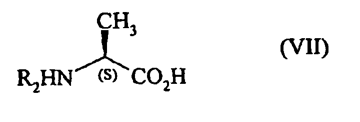 dans laquelle R₂ est tel que défini dans la formule (I),
dans un solvant organique tel que, par exemple, le tétrahydrofurane ou l'acétate d'éthyle,
en présence d'une quantité de dicyclohexylcarbodiimide comprise entre 1 et 1,2 mole par mole de composé de formule (VI) utilisé, d'une quantité de triéthylamine comprise entre 1 et 1,2 mole par mole de composé de formule (VI) utilisé, et en présence éventuelle de 1-hydroxybenzotriazole,
à une température comprise entre 20 et 50°C,
pour conduire après isolement puis recristallisation au composé de formule (VIII) : 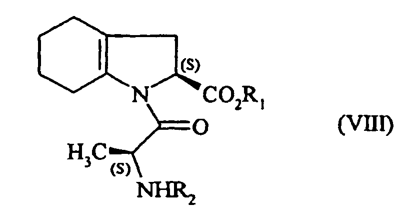 dans laquelle R₁ et R₂ sont tels que définis précédemment,
que l'on hydrogène en présence d'un catalyseur tel que le palladium, le platine, le rhodium ou le nickel,
sous une pression d'hydrogène comprise entre 1 et 30 bars, pour conduire, après éventuelle déprotection ou reprotection de la fonction acide, au composé de formule (I).

2. Procédé de synthèse selon la revendication 1, **caractérisé en ce que** la pression d'hydrogène de la réaction d'hydrogénation est comprise entre 1 et 10 bars.

3. Procédé de synthèse selon la revendication 1, permettant l'obtention du dérivé de formule (I) dans laquelle R₁ représente un atome d'hydrogène et R₂ représente un groupement tert-butyloxycarbonyle.

4. Procédé de synthèse du perindopril ou de ses sels pharmaceutiquement acceptables à partir d'un composé de formule (I), **caractérisé en ce que** ledit composé de formule (I) est obtenu selon le procédé de la revendication 1.

5. Composé de formule (VIII) : 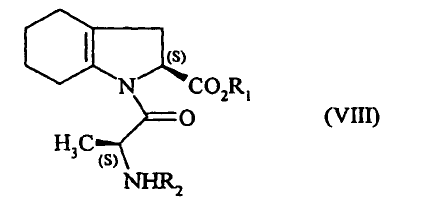 dans laquelle R₁ représente un atome d'hydrogène ou un groupement alkyle (C₁-C₆) linéaire ou ramifié ou benzyle, et R₂ représente un groupement protecteur de la fonction amine.

## Patentansprüche

1. Verfahren zur Synthese der Verbindungen der Formel (I): 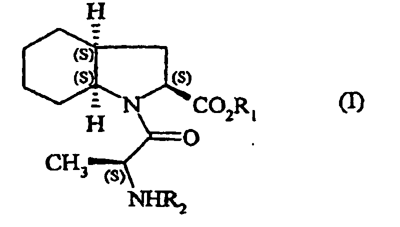 in der R₁ ein Wasserstoffatom oder eine geradkettige oder verzweigte (C₁-C₆)-Alkylgruppe oder eine Benzylgruppe und R₂ eine Schutzgruppe für die Aminfunktion bedeuten,
**dadurch gekennzeichnet, daß** man 1-(1-Cyclohexen-1-yl)-pyrrolidin der Formel (III): 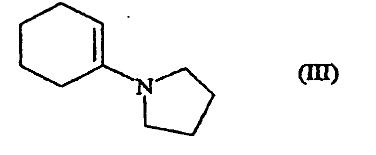 mit dem Serin-Derivat der Formel (IV): 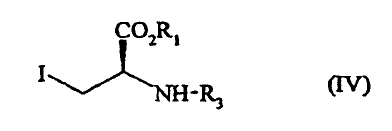 in der R₁ die bezüglich der Formel (I) angegebenen Bedeutungen besitzt und R₃ eine Schutzgruppe für die Aminfunktion darstellt, umsetzt,
zur Bildung der Verbindung der Formel (V): 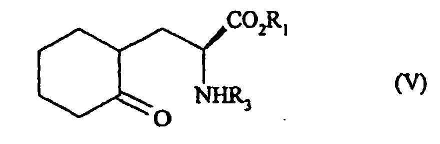 in der R₁ und R₃ die oben angegebenen Bedeutungen besitzen,
von der man die Schutzgruppe der Aminfunktion abspaltet, bevor man die Cyclisierung, gefolgt von der Dehydratisierung, bewirkt zur Bildung der Verbindung der Formel (VI): 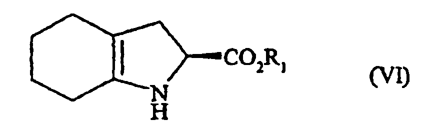 in der R₁ die oben angegebenen Bedeutungen besitzt,
welche man in einem organischen Lösungsmittel, wie beispielsweise Tetrahydrofuran oder Ethylacetat,
in Gegenwart einer Menge Dicyclohexylcarbodiimid zwischen 1 und 1,2 Mol pro Mol der verwendeten Verbindung der Formel (VI), einer Menge Triethylamin zwischen 1 und 1.2 Mol pro Mol der verwendeten Verbindung der Formel (VI) und gegebenenfalls in Gegenwart von 1-Hydroxybenzotriazol
bei einer Temperatur zwischen 20 und 50°C mit dem Alanin-Derivat der Formel (VII) : 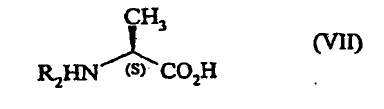 in der R₂ die bezüglich der Formel (I) angegebene Bedeutung besitzt, umsetzt,
so daß man nach der Isolierung und der Umkristallisation die Verbindung der Formel (VIII) erhält: 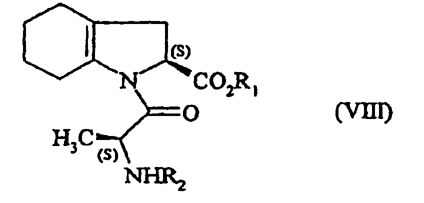 in der R₁ und R₂ die oben angegebenen Bedeutungen besitzen,
welche man in Gegenwart eines Katalysators, wie Palladium, Platin, Rhodium oder Nickel,
bei einem Wasserstoffdruck zwischen 1 und 30 bar hydriert, so daß man nach der eventuellen Abspaltung der Schutzgruppe oder der Wiedereinführung der Schutzgruppe der Säurefunktion die Verbindung der Formel (I) erhält.

2. Syntheseverfahren nach Anspruch 1, **dadurch gekennzeichnet, daß** der Wasserstoffdruck bei der Hydrierreaktion zwischen 1 und 10 bar liegt.

3. Verfahren nach Anspruch 1 zur Synthese des Derivats der Formel (I), in der R₁ ein Wasserstoffatom und R₂ eine tert.-Butyloxycarbonylgruppe bedeuten.

4. Verfahren zur Synthese von Perindopril oder von seinen pharmazeutisch annehmbaren Salzen ausgehend von einer Verbindung der Formel (I), **dadurch gekennzeichnet, daß** die Verbindung der Formel (I) nach dem Verfahren gemäß Anspruch 1 hergestellt wird.

5. Verbindung der Formel (VIII): 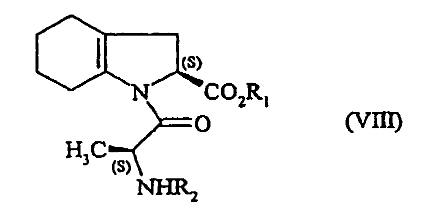 in der R₁ ein Wasserstoffatom oder eine geradkettige oder verzweigte (C₁-C₆)-Alkylgruppe oder eine Benzylgruppe und R₂ eine Schutzgruppe für die Aminfunktion bedeuten.

## Claims

1. Process for the synthesis of compounds of formula (I) : 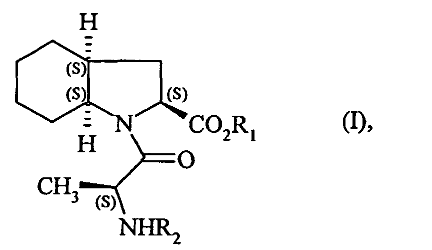 wherein R₁ represents a hydrogen atom or a linear or branched (C₁-C₆)alkyl or benzyl group and R₂ represents a protecting group for the amine function,
**characterised in that** 1-(1-cyclohexen-1-yl)-pyrrolidine of formula (III) : 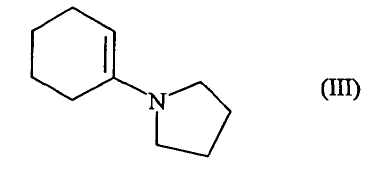 is reacted with the serine compound of formula (IV) : 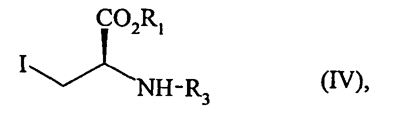 wherein R₁ is as defined for formula (I) and R₃ represents a protecting group for the amine function,
to yield the compound of formula (V) : 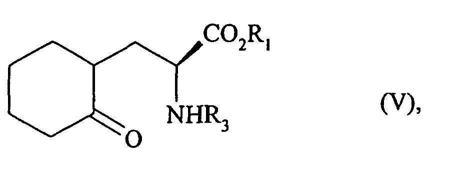 wherein R₁ and R₃ are as defined hereinbefore,
the amine function of which is deprotected before cyclisation is carried out, followed by dehydration, to yield the compound of formula (VI) : 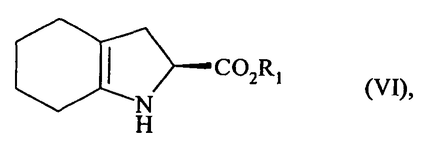 wherein R₁ is as defined hereinbefore,
which is reacted with the alanine compound of formula (VII) : 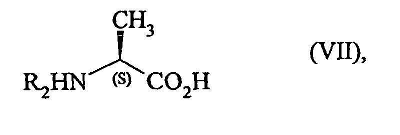 wherein R₂ is as defined for formula (I),
in an organic solvent such as, for example, tetrahydrofuran or ethyl acetate,
in the presence of an amount of dicyclohexylcarbodiimide of from 1 to 1.2 mol per mol of compound of formula (VI) used and an amount of triethylamine of from 1 to 1.2 mol per mol of compound of formula (VI) used and optionally in the presence of 1-hydroxybenzotriazole,
at a temperature of from 20 to 50°C,
to yield, after isolation and then recrystallisation, the compound of formula (VIII) : 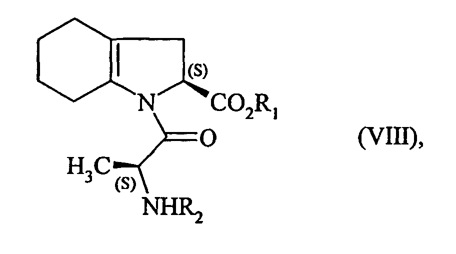 wherein R₁ and R₂ are as defined hereinbefore,
which is hydrogenated in the presence of a catalyst such as palladium, platinum, rhodium or nickel,
under a hydrogen pressure of from 1 to 30 bars, to yield, after optional deprotection or reprotection of the acid function, the compound of formula (I).

2. Synthesis process according to claim 1, **characterised in that** the hydrogen pressure in the hydrogenation reaction is from 1 to 10 bars.

3. Synthesis process according to claim 1, allowing the compound of formula (I), wherein R₁ represents a hydrogen atom and R₂ represents a tert-butoxycarbonyl group, to be obtained.

4. Process for the synthesis of perindopril or pharmaceutically acceptable salts thereof starting from a compound of formula (I), **characterised in that** the said compound of formula (I) is obtained according to the process of claim 1.

5. Compound of formula (VIII) : 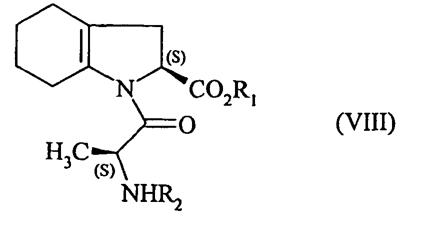 wherein R₁ represents a hydrogen atom or a linear or branched (C₁-C₆)alkyl or benzyl group and R₂ represents a protecting group for the amine function.
